# EUROPEAN PATENT APPLICATION

(11) **EP 3 756 477 A1**
(43) Date of publication of application: **30.12.2020**
(21) Application number: 20181129.6
(22) Date of filing: 19.06.2020
(51) Int. Cl.: A23L 33/18, A23L 33/16, A23L 33/165, A23L 33/00, A61P 19/10, A61K 38/01, A23B 4/027, A61K 33/06, A61K 33/24, A61K 33/26, A61K 33/30, A61K 33/32, A61K 33/34, A61K 38/04, A61K 38/05, A61K 38/06, A61K 38/07, A61K 38/08

(54) **NANO COLLAGEN PEPTIDE CHELATE MINERAL AND METHOD FOR PREPARING THE SAME**

(30) Priority: 28.06.2019 KR 20190078358
(71) Applicant: RE & C Bio. Co., Ltd., Gyeonggi-do 14057 (KR)
(72) Inventor: CHOI, Moon Bum, 14076 Gyeonggi-do (KR); JANG, Boo Sik, 28116 Chungcheongbuk-do (KR); YUN, Ji Suk, 14044 Gyeonggi-do (KR)
(74) Representative: Frenkel, Matthias Alexander

(57) **Abstract**

The present disclosure relates to a nano collagen peptide chelate mineral that can be used in food, medicine, quasi-drugs, cosmetics or feed, and method for preparing the same, and the purpose of the present disclosure is to provide a nano collagen peptide chelate mineral that has excellent product preservation or stability, and also excellent absorption rate of not only collagen peptide in skin or human body but also excellent absorption rate of chelated mineral in the collagen peptide, and method for preparing the same.

## Description

### 1. Field

The present disclosure relates to a nano collagen peptide chelate mineral and method for preparing the same.

### 2. Background

Calcium (Ca), the most abundant mineral in the body, is mostly used to make bones and teeth, but about 1% is dissolved in the blood and circulates to regulate the functions of muscles and nerves and help blood clotting. However, if too much calcium in the blood builds up in certain tissues or organs, calcification occurs. Of all the minerals, calcium exists most abundantly in the body, accounting for about 900 to 1200g, that is, 1.5 to 2.0% of an adult's weight. 99% of the calcium exists in the bones and teeth, and the remaining 1% exists in the blood, extracellular fluid, muscles and the like.

Important physiological actions of calcium include blood clotting, muscle contraction and relaxation, regular beating of the heart, secretion of neurotransmitters, activation of enzymes, movement of villi, phagocytosis of white blood cells, division of cells, and metabolism of various nutrients, etc. Further, calcium acts as a regulator of mass transport through cell membranes. Calcium plays a role not only in the bone density, but also in weight control, diabetes, and prevention of colon cancer. In other words, when on a diet to lose weight, taking calcium around 1,000mg or more will have a greater weight control effect, and it is reported that high calcium intake groups have a reduced risk of developing diabetes and colorectal cancer compared to low calcium intake groups.

Calcium is absorbed mostly by active transport in the upper small intestines and by simple diffusion in the lower small intestines. The absorption rate of calcium is known to be around 60% in breastfeeding infants, 40% in childhood and adolescence, and 30% in adulthood. Further, the absorption rate of calcium is affected by the dietary ingredients you consume. That is, protein, vitamin D, lactose, and peptides promote calcium absorption, but fat, dietary fiber, phosphoric acid, oxalic acid, and phytic acid inhibit calcium absorption.

Meanwhile, collagen is also called light protein, and is widely distributed in multicellular animals such as invertebrates and vertebrates, and it is the most widely found light protein in quantity. Accounting for about one third of all proteins in mammals, collagen is a light protein with a very strong fiber-like tension that makes up the connective tissue of animals, and that transfers power from the tendon or ligament without loss. The basic structural unit of collagen is tropocollagen with a molecular weight of about 300,000. This molecule has a triple stranded polypeptide chain with a right-hand twist. Tropocollagen molecules assemble to form collagen fibers, and each molecule is arranged in displacement by one quarter in the axial direction to form a unique stripe pattern with an interval of 64 nm. With the growth of animal, a bridge-like structure is formed between the molecules, making it insoluble. However, when collagen is boiled for a long time in hot water, dilute acid or dilute alkali, it turns into gelatin, an inducible protein that dissolves in water.

Collagen peptide is a kind of fibrous protein composed of glycine, proline, hydroxyproline, glutamic acid, etc. It is a light protein having the shape of a thin, elongated band in which about 1,000 amino acids are gathered. It is mainly present in the membranes, joint cartilage, corneas of the eyes, bones and skin surrounding the organs in the human body, and especially, it plays a very important role as a component of the dermal layer inside the skin.

Providing firmness to the skin, resistance and binding force of tissues, and support for cell adhesion are knowns as the main functions of collagen. It is known that collagen is thinned due to aging and photoaging due to ultraviolet irradiation, and this phenomenon is closely related to the formation of wrinkles on the skin.

Rising levels of income and entrance into aging societies have led to extensive research on skin aging, and functions of collagen on skin are being revealed. Among them, due to the claim that as collagen synthesis is promoted and collagen metabolism becomes active, the components of the dermis matrix will increase, thus providing effects of anti-wrinkle, elasticity enhancement, skin strengthening and wound healing, many products using collagen synthesis accelerators such as retinoic acid, Transforming Growth Factor (TGF), animal-derived placenta, betulinic acid, and chlorella extract as cosmetic compositions for strengthening skin have been released, but did not receive much attention. In addition, many collagen products are in the market that contain collagen as a cosmetic composition for protection of skin, but it has been pointed out that it is difficult to expect the effect of moisturizing the skin from cosmetics made to be applied to the surface of the skin since it is difficult for the polymer collagen to be absorbed into the percutaneous layer.

Recently, collagen peptides are increasingly being used in health food, surgical and ophthalmic therapeutics, and cosmetic additives. Meanwhile, in the perspective of food science, pharmaceutical and cosmetic science, in these collagen peptides, stability of the products and their absorption rates in the skin and in the body are increasingly considered important.

With this in mind, the present disclosure aims to provide a chelate collagen peptide in which mineral components that are necessary in the human body, such as calcium, and collagen peptides are chelated, and a method for preparing the same.

### [Prior Art Literature]

### [Patent Literature]

Korean Laid-open Patent Publication No. 10-2017-0093694 (August 16, 2017)
Korean Registered Patent Publication No. 10-1254403 (April 15, 2013)
Korean Registered Patent Publication No. 10-0488913 (May 11, 2005)

### SUMMARY

The present disclosure relates to a nano collagen peptide chelate mineral that can be used in food, medicine, quasi-drugs, cosmetics or feed, and a method for preparing the same, and particularly, to a nano collagen peptide chelate mineral with excellent product preservation or stability, and excellent absorption rate of not only the peptide into the skin or body, but also the absorption rate of the mineral chelated in the nanoscale sized collagen peptide, and a preparation method of the same.

The present disclosure provides a nano collagen peptide chelate mineral preparation method including: 1) forming a nanoscale sized collagen peptide by adding an enzyme to collagen to hydrolyze the collagen; and
2) forming a collagen peptide chelate by adding a mineral to the collagen peptide.

Further, the present disclosure provides a nano collagen peptide chelate mineral prepared by the described preparation method of the present disclosure and represented by [Chemical Formula 1] below.

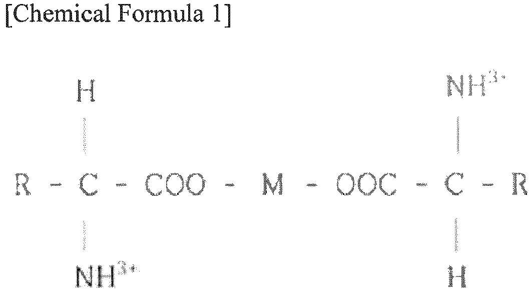

(R representing a side chain that determines a type of amino acid, and M being one or more type selected from a group consisting of Ca, Cu, Zn, Fe, Se, Cr, Mg, Mn and Co.)

When used in food, the nano collagen peptide chelate mineral of the present disclosure supplies calcium in the body to help bones and teeth formation, strengthens the alveolar bones to make the gums healthy, maintains nerve and muscle function, reduces the risk of osteoporosis, strikes a balance between the potential difference of the cell membranes, being helpful for vascular disease and heart disease. Further, the nano collagen peptide chelate mineral of the present disclosure not only supplies high quality protein but is also effective in suppressing aging caused by ultraviolet rays and improving wrinkles, and when used in cosmetics, it maintains the moisturizing properties by increasing the water content in the skin, and provides wrinkle improvement effects.

Moreover, it has effects of excellent product preservation or stability, and excellent absorption rate of not only the peptide into the skin or body, but also the absorption rate of the mineral chelated in the nanoscale sized collagen peptide.

Specifically, the nano collagen peptide chelate mineral of the present disclosure uses collagen, which plays an important role in bone formation, regeneration and recovery, as an excipient, and as the molecules can be divided into nanoscale sized molecules, its bioavailability can be maximized compared to conventional mineral amino acid chelates.

Further, the nano collagen peptide chelate mineral of the present disclosure can tightly bind the collagen peptide and mineral through coordination covalent bonds, thereby preventing anion binding and competitive absorption to maximize the amount of mineral reaching the small intestines. Moreover, since it can be absorbed without being ionized by an enzyme, it can be absorbed not only from the upper end of the small intestines but also from the entire small intestines. In addition, it is possible to secure fat-soluble properties through binding with collagen peptides, whereby it can be absorbed in the entire cell membranes, therefore maximizing the amount of minerals being absorbed from the small intestines to the cells. Further, it can minimize the effect of vitamin D, so that a more stable calcium absorption can be achieved.

### DETAILED DESCRIPTION

Hereinafter, the present disclosure will be described in more detail for better understanding of the present disclosure. Here, the terms or words used in the present specification and claims set should not be interpreted as being limited to ordinary or dictionary meanings, but should be interpreted to have the meaning and concept that are consistent with the technical idea of the present disclosure based on the principle that the inventor can define the concept of the terms appropriately in order to explain their invention in the best way possible.

The present disclosure relates to a nano collagen peptide chelate mineral that can be used in food, medicine, quasi-drugs, cosmetics or feed, and method for preparing the same, and the purpose of the present disclosure is to provide a nano collagen peptide chelate mineral having effects of excellent product preservation or stability, and excellent absorption rate of not only the peptide into the skin or body, but also the absorption rate of the mineral chelated in the nanoscale sized collagen peptide.

Specifically, the present disclosure provides a nano collagen peptide chelate mineral preparation method including 1) forming a nanoscale sized collagen peptide by adding an enzyme to collagen to hydrolyze the collagen; and
2) forming a collagen peptide chelate by adding a mineral to the collagen peptide.

Collagen is also called light protein, and is widely distributed in multicellular animals such as invertebrates and vertebrates, and it is the most widely found light protein in terms of quantity. It is mainly present in the human body's membranes covering the organs, joint cartilage, corneas of the eyes, bones and skin, and especially, it plays a very important role as a component of the dermal layer inside the skin. Providing firmness to the skin, resistance and binding force of tissues, and support for cell adhesion are knowns as the main functions of collagen.

Collagen can be largely classified into animal collagen such as cow skin, pig skin, vegetable collagen such as fruits, vegetables, seeds, nuts, mushrooms, and marine collagen such as algae, fish, fish scales, and fish skin. The collagen used in preparing the chelate collagen peptide of the present disclosure is characterized to be one or more selected from a group consisting of the animal collagen, vegetable collagen and marine collagen.

The chelate collagen peptide of the present disclosure is characterized, first of all, to 1) form a nanoscale sized collagen peptide by adding an enzyme to collagen to hydrolyze the collagen (Step 1) in the present disclosure is also called 'collagen nano step').

Collagen is a fibrous protein found in most animals, especially mammals. It accounts for most of all connective tissues in the body, including skin and cartilage. Collagen has a triple helix structure in which three polypeptide chains are twisted, and the average molecular weight of collagen is quite large: 300,000 Dalton (Da).

In order for the collagen having such a large molecular weight to be absorbed into the body, the process of digestion in the stomach must be preceded. Thus, the method for preparing the nano collagen peptide chelate mineral of the present disclosure includes the step of adding an enzyme for rapid absorption in the body to hydrolyze the collagen thereby decomposing it into nanoscale collagen peptides.

The average molecular weight of the collagen peptide of the present disclosure is 200 Da to 600 Da, and more particularly, 300 Da to 400 Da, and it's size is 0.1 nm to 10 nm, and more particularly, it is nanosized to be 1 nm to 5 nm, and therefore, due to its small molecular weight and small size, it shows the effect of being quickly absorbed into the body or skin even before the digestion process.

Here, the enzyme used in the collagen nano step is a proteolytic enzyme, which is characterized to include one or more type selected from a group consisting of collagenase, trypsin, papain, pepsin and alcalase, and the hydrolysis reaction for degrading collagen to collagen peptide is performed at 50°C to 70°C, and more particularly at 50°C to 60°C for 2 to 24 hours.

When the hydrolysis reaction occurs under the conditions of temperature and time in the above range, it is possible to obtain a collagen peptide with appropriate molecular weight and size, having a high absorption rate in the body.

After the collagen nano step, the present disclosure is characterized to include 2) forming a collagen peptide chelate by adding a mineral to the collagen peptide (step 2 of the present disclosure is also called 'collagen peptide chelate step').

The mineral used in the present disclosure is one or more kind of hydroxide or sulfur oxide selected from a group consisting of Ca, Cu, Zn, Fe, Se, Cr, Mg, Mn and Co, and stabilization is obtained by performing ion-bonding in the form of adding minerals instead of H ions of the COOH group, while conducting covalent bonding using the terminal group of the amino acid from NH²⁺ to NH³+

The mineral used is 1 to 20 parts by weight based on 100 weight of collagen peptide, and within this range, chelate collagen peptide can be obtained with high purity without unreacted minerals.

Chelate refers to a bond formed when a ligand having a plurality of coordination bonds with a metal ion coordinates with a metal, or a complex produced by the bond. In the present disclosure, a non-covalent electron pair that could not participate in the covalent bond of the amino group bound to the alpha carbon of the amino acid forms a perfect coordination bond with the mineral to form the collagen peptide chelate.

The present disclosure can improve the product preservation or stability through the chelation of these collagen peptide and mineral, and also provide an effect of improving the absorption rate of collagen peptide and mineral into the body or skin.

Thereafter, the nano collagen peptide chelate mineral preparation method of the present disclosure may further include one or more step selected from a group consisting of refining, sterilizing and drying step.

Through the refining step, a nano collagen peptide chelate mineral with high purity can be prepared. The refining step may be performed by filtrating and separating the collagen peptide having high molecular weight or large size, and unreacted enzyme and minerals, and byproducts, that have not been hydrolyzed into appropriate molecular weight and size by the collagen nano step 1).

Further, through physical or chemical processing, microorganisms that are harmful to the human body can be removed. It can be added to a heat-resistant appliance (glassware, ceramics, some metal products, etc.) and heated at 70°C to 90°C for 1 to 5 hours to dry and sterilize harmful microorganisms and bacteria.

Moreover, depending on the type of final product, a drying step using a lyophilizer or spray dryer may be additionally performed.

The present disclosure provides a nano collagen peptide chelate mineral that may be prepared by the nano collagen peptide chelate mineral preparation method described above, and that is represented by the Chemical Formula 1 below.

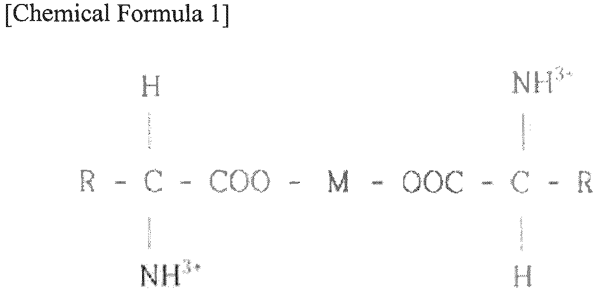

(R representing a side chain that determines a type of amino acid, and M being one or more type selected from a group consisting of Ca, Cu, Zn, Fe, Se, Cr, Mg, Mn and Co.)

The nano collagen peptide chelate mineral of the present disclosure may be used in food, medicine, quasi-drugs, cosmetics or feed, and its formulation may be in powder, tablet, granule, pill and the like.

The nano collagen peptide chelate mineral of the present disclosure can be absorbed quickly into the body or skin within one hour, more particularly, within thirty minutes, together with the chelated mineral, and thus when taken or applied for a long period of time, it can show excellent effects in improving skin moisturizing, elasticity and wrinkles compared to other collagen products.

Hereinbelow, embodiments of the present disclosure will be described in detail so as to be easily implemented by a person with ordinary knowledge in the art that the present disclosure pertains to. However, the present disclosure may be be embodied in various different forms, and thus is not limited to the embodiments described herein.

### Example 1

Marine collagen of fish shells and jellyfish was washed with purified water to remove impurities, and for 1 kg of raw material, 4 liters of purified water was added, and then crushed to 50 µm by a chopper. Next, the raw material deodorized by passing through ozone was placed in a 0.5% citric acid solution and hydrolyzed at 55°C for 24 hours by adding 0.5 w% of pepsin protease, to obtain collagen peptide of 300 Da to 400 Da, with size of 1 nm to 5 nm.

For preparation of the nano collagen peptide chelate mineral described above, a weight ratio of calcium was added by weight parts so that the calcium content is 1 to 20 parts by weight using calcium carbonate compared to 100 weights of collagen peptides, followed by a chelation reaction while hydrolyzing at 55°C, and then dried using a spray dryer, thereby preparing Ca-collagen peptide chelate complex powder.

### Comparative Example 1

Collagen peptide powder was prepared in the same manner as in Example 1 described above, except that calcium carbonate was not added.

### Comparative Example 2

Calcium carbonate powder was prepared.

### Experimental Example 1

Using Example 1 and Comparative Example 1 described above, food anti-aging and preservation improvement effect were experimented.

### 1-1) Food Anti-aging Experiment

Using the collagen peptide described above, it was decided to try and grasp its anti-aging effect on rice, gimbap, white bread, tofu, and agar jelly. The anti-aging effect was experimented by mixing or evenly sprinkling 0.05 to 0.2% by weight of collagen peptide in cooked food or ingredients, and after 24 to 72 hours at room temperature, comparison was made through visual observation and sensory testing of the degree of hardening and discoloration and the like during storage, and the results (S; very good, A; good, B; normal, C; poor) are as shown in Table 1 below.

**[Table 1]**

| Division | Application Examples | | | | |
|---|---|---|---|---|---|
| | Rice | Gimbap | White bread | Tofu | Agar jelly |
| Example 1 | S | S | S | A | S |
| Comparative example 1 | C | B | C | C | C |

As shown in Table 1, it was found that the food to which the Ca-collagen peptide chelate complex of Example 1 was added, did not harden even when stored for a long period of time, and the discoloration was less.

### 1-2) Preservation Improvement Effects Experiment

In order to grasp the preservation improvement effects of the collagen peptide when added to porridge and rice, 0.05 to 0.2% by weight of collagen peptide was mixed or evenly sprinkled with rice porridge, abalone porridge, mixed grain rice, and brown rice, then after 24 to 72 hours at room temperature, rotting smell (odor) was grasped, and the results are as shown in Table 2.

Meanwhile, in order to quantitatively determine the preservation improvement effects, the total number of bacteria in Example 1 and Comparative Example 1 were compared. For the total number of bacteria, homogenized water obtained by adding 112.5 ml of peptone water to 12.5g of specimen was used as the experimental solution. Then, the experimental solution was diluted to 1ml, and for each step, this 1ml was dispensed aseptically into three sterile Petri dishes, and then 15 ml of plate count agar (PCA) maintained at 46 to 47°C was added aseptically and mixed with the experimental solution. After solidification, the medium was cultivated at 37°C for 24 hours, and plates that produced 50 to 400 colonies per plate were selected to obtain cfu per g of counting samples of colonies.

**[Table 2]**

| Division | Application Examples | | | | Notes |
|---|---|---|---|---|---|
| | Rice porridge | Abalone porridge | Mixed grain rice | Brown rice | |
| Example 1 | 4.82±0.04 | 5.83±0.06 | 5.75±0.04 | 5.87±0.04 | Unit of the total number of bacteria shown in Log/cfu/g |
| Comparative example 1 | 10.98±0.06 | 13.27±0.05 | 9.46±0.05 | 10.08±0.06 | |

As can be seen from Table 2, based on the result of measurement of the total number of bacteria, food in which the Ca-collagen peptide chelate complex of Example 1 was added showed far more excellent preservation improvement effects.

### Experimental Example 2

Using the collagen peptide of Example 1 and Comparative Example 1 described above, nutrition cream was prepared as shown in Table 3 below and experimented to observe the skin beauty effects.

**[table 3]**

| Composition | Composition Ratio (Weight %) | |
|---|---|---|
| | Example 1 | Comparative Example 1 |
| Collagen Peptide | 10.0 (Chelate Complex) | 10.0 |
| Glvcerin | 7.0 | 7.0 |
| Propylene Glycol | 5.0 | 5.0 |
| Polyvinyl Alcohol | 14.0 | 14.0 |
| Ethanol | 9.0 | 9.0 |
| Polyoxyethylene oleyl oil | 1.5 | 1.5 |
| Paraoxybenzoic acid methyl | 0.1 | 0.1 |
| Flagrant | Appropriate amount | Appropriate amount |
| Purified water | 53.4 | 53.4 |

### 2-1) Skin Water Content Measurement

In order to grasp the skin moisturizing effect, Example 1, Comparative Example 1 and purified water were used as experimental specimens using the measuring device (IMPEDANCE METER, SKICON-200 IBS JAPAN), and on the inner side of the right arm of a 20-year-old woman, about 1 ml of each sample was applied, and the after thirty minutes, the remaining liquid was removed, and electrical conductivity of the skin was measured at every elapsed time, and the results are as shown in Table 4 below.

**[Table 4]**

| Division (Unit: Ω) | 0 (sec) | 30 (sec) | 60 (sec) | 150 (sec) | 240 (sec) | 330 (sec) | 420 (sec) | 510 (sec) |
|---|---|---|---|---|---|---|---|---|
| Example 1 | 140 | 1600 | 650 | 420 | 340 | 320 | 310 | 300 |
| Comparative Example 1 | 130 | 1100 | 270 | 140 | 130 | 125 | 120 | 110 |
| Purified Water | 80 | 800 | 180 | 120 | 115 | 110 | 80 | 60 |

As can be seen from Table 4, the moisturizing properties were shown in the order of Example 1>Comparative Example 1>Purified Water. Through this, it was found that the nutrition cream to which the Ca-collagen peptide chelate complex of Example 1 was added had excellent moisturizing properties.

### 2-2) Wrinkle Improvement Experiment

Thirty women in their 50s were randomly divided into a first group and a second group, and for the first group, the cream of Example 1 was applied around the eyes twice a day every morning/evening for 2 months, and for the second group, the cream of the Comparative Example 1 was applied around the eyes twice a day every morning/evening for 2 months, to see whether the wrinkles around eyes improved, and the results are as shown in Table 5 below.

**[Table 5]**

| Division | No improvement | Slight improvement | Medium improvement | Significant improvement |
|---|---|---|---|---|
| First Group | 0 | 1 | 3 | 11 |
| Second Group | 0 | 4 | 5 | 6 |

There was some wrinkle improvement around the eyes of the second group to which the cream of Comparative Example 1 was applied, but in the first group to which the cream of Example 1 was applied, eleven people showed significant wrinkle improvement. Through this, it was confirmed that the cream of Example 1 has excellent wrinkle improvement effects.

### Experimental Example 3

Bioavailability improvement effect was experimented using the powder of Example 1 and Comparative Example 2.

### 3-1) Bioavailability Improvement Effects Experiment 1

Five rats of six weeks of age, each having a body weight of about 180g, were grouped into experimental and control groups. For the experimental group, the powder of Example 1 was dissolved in a 0.5% CMC-Na solution, and was forcibly administered orally using zones by 10ml at a time to be 100 mg/kg. For the control group, the powder of Comparative Example 2 was dissolved in 0.5% CMC-Na solution, and was forcibly administered orally using zones by 10ml at a time to be 100mg/kg.

After one week since administration of specimen, 500ml of blood was taken from each rat in the groups, and centrifuged at 2500rpm, and then only the upper layer liquid portion was taken to be used as the blood specimen of each group. The blood specimen was used to measure the absorption rate of calcium and the amount of calcium in the serum, and then it was measured whether there was any growth improvement. The results are as shown in Table 6 below.

**[Table 6]**

| Measurement Items | Control group | Experimental group |
|---|---|---|
| Diet intake (g/d) | 12.27 | 12.55 |
| Initial weight (g) | 203.46 | 204.47 |
| Final weight (g) | 251.25 | 256.53 |
| Body weight gain (g) | 47.79 | 52.05 |
| Ca intake (mg/d) | 50.25 | 51.31 |
| Foecal Ca (mg/d) | 34.13 | 18.38 |
| Apparent absorption (%) | 32.09 | 64.18 |
| Ca (mmol/L) | 2.36 | 2.58 |
| P (mmol/L) | 2.17 | 2.14 |
| ALP (U/L) | 185.88 | 177.13 |

According to Table 6, compared to the control group, the rats in the experimental group showed greater increase in the weight, and therefore, it shows that the powder in Example 1 has the growth improvement effects. Further, compared to the control group, the rats in the experimental group showed twice or more calcium absorption rate, and therefore, it shows that the powder of Example 1 is helpful to improve the calcium absorption rate. Moreover, compared to the control group, the rats in the experimental group had higher contents of calcium in the serum and lower contents of Alkaline Phophatase (ALP) in the serum, and therefore, it shows that the powder of Example 1 is effective in improving the calcium absorption rate.

### 3-2) Bioavailability Improvement Effects Experiment 2

Rats were grouped into experimental and control groups as in 3-1) Experiment described above, and the length, weight, and bone density of the thigh bone of the rats of each group were measured, and then the calcium content, maximum load, elastic load, and strength of the thigh bone were measured. The results are as shown in Table 7 below.

**[Table 7]**

| Measurement Items | Control group | Experimental group |
|---|---|---|
| Length (cm) | 33.24 | 33.8 |
| Weight (mg) | 856.33 | 873.31 |
| BMD, midshaft (g/cm2) | 186.25 | 217.5 |
| Ca content (mg/g) | 228.46 | 254.75 |
| Maximum load (N) | 174.04 | 199.93 |
| Elastic load (N) | 141.71 | 170.43 |
| Stiffness (N/mm) | 155.99 | 180.59 |

According to Table 7, compared to the control group, the rats in the experimental group showed higher measurements in all of the bone density, calcium content, maximum load, and strength, and therefore, it shows that the powder of Example 1 strengthens bones and reduces the risk of developing osteoporosis. Moreover, changes in the systemic bone density and lumbar bone density in the control and experimental group were measured while orally administering each powder to each group for three months, and the results showed that the systemic bone density of the control group decreased by 1.2% whereas the systemic bone density of the experimental group increased by 1%. In addition, the lumbar bone density of the control group increased by 0.2% while the lumbar bone density of the experimental group increased by 0.9%. This shows that the powder of Example 1 helps improve not only the systemic bone density but also the lumbar bone density.

### 3-3) Bioavailability Improvement Effect Experiment 3

Rats were grouped into experimental and control groups as in 3-1) Experiment described above, and the total calcium content and total calcium ion content in the serum of the rats of each group were measured. The results are as shown in Table 8 below.

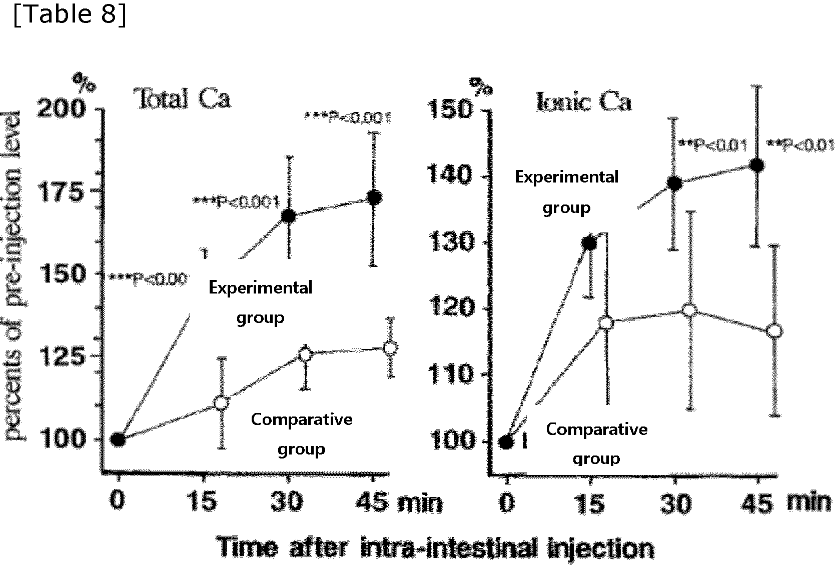

According to Table 8, compared to the rats to which the powder of Comparative Example 2 was administered, the total calcium content and total calcium ion content in the serum of the rats to which the powder of Example 1 was administered were higher, and therefore, it shows that the powder of Example 1 is helpful to the calcium absorption rate.

### 3-4) Bioavailability Improvement Effects Experiment 4

Female rats to which calcium carbonate (calcium 1%) was administered were prepared as a control group, and ovariectomized rats to which low calcium (calcium 0.03%) was administered were prepared as a experimental group. Thereafter, the bone volume of the control and experimental group were measured, and then three months after, the bone volume of the experimental group was measured after orally administering the powder of Example 1 to the experimental group. The results are as shown in Table 9 below.

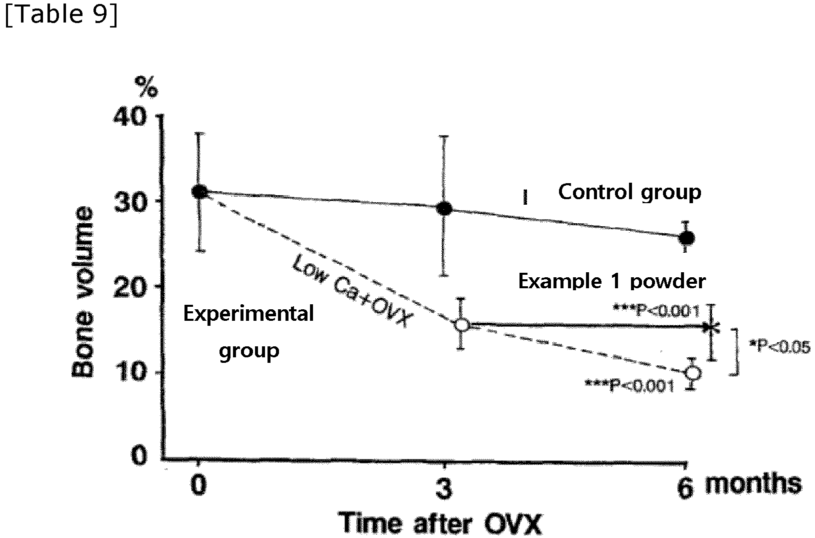

According to Table 9, the bone volume of the experimental group continued to decrease for the first three months, but after administering the powder of Example 1 three months after, the bone volume did not decrease and was maintained. This shows that the powder of Example 1 was helpful in improving the calcium absorption rate.

### 3-5) Bioavailability Improvement Effects Experiment 5

Climacteric women of forty-five to fifty-five years of age were grouped into experimental and control groups. For the experimental group, the powder of Example 1 made in soft capsules was administered, and for the control group, the powder of Comparative Example 2 also made in soft capsules were administered. The Bone-Specific Alkaline Phosphatase (BAP), which is an indicator for bone formation, the tartrate-resistant acid phosphatase isoform 5b (TRAP5b), which is an indicator for bone reabsorption, and Bone Mineral Density (BMD), were measured while continuing the administration for a long period of twelve months. The results are as shown in Table 10 below.

**[Table 10]**

| | Control Group | | | Experimental Group | | |
|---|---|---|---|---|---|---|
| | Baseline | Six months | Twelve months | Baseline | Six months | Twelve months |
| BAP (U/L) | 30.63 | 26.80 | 29.03 | 30.85 | 30.99 | 30.72 |
| TRAP5b (U/L) | 3.89 | 3.27 | 4.94 | 3.65 | 3.04 | 3.87 |
| Sclerostin (ng/mL) | 0.56 | 0.51 | 0.56 | 0.61 | 0.49 | 0.51 |
| BAP/TRAP5b | 0.09 | 8.53 | 6.91 | 10.08 | 12.46 | 9.59 |
| BMD-body (g/cm2) | 1.049 | 1.037 | 1.026 | 1.070 | 1.070 | 1.067 |
| BMD-hip (g/cm2) | 0.817 | 0.812 | 0.805 | 0.878 | 0.872 | 0.875 |

According to Table 10, the long term experiment results show that the total bone density of the control group decreased whereas the bone density of the experimental group was maintained. In addition, it shows that the femoral bone density of the experimental group had a significant increase. Further, compared to the control group, the experimental group showed a higher level of bone-forming substance and a lower level of substance that interferes with bone-forming (sclerostin).

The above description of the present disclosure is for illustrative purpose, and any person having ordinary knowledge in the art to which the present disclosure pertains will understand that the present disclosure can be easily modified in other particular forms without changing the technical spirit or essential features of the present disclosure. Therefore, it should be understood that the embodiments described above are illustrative in all respects and not restrictive.

## Claims

1. A nano collagen peptide chelate mineral preparation method comprising:
1) forming a nanoscale sized collagen peptide by adding an enzyme to collagen to hydrolyze the collagen; and
2) forming a collagen peptide chelate by adding a mineral to the collagen peptide.

2. The nano collagen peptide chelate mineral preparation method, according to claim 1, wherein the collagen peptide has an average molecular weight of 200 Da to 600 Da.

3. The nano collagen peptide chelate mineral preparation method, according to claim 1 or 2,
wherein the collagen peptide has a size of 0.1 nm to 10 nm.

4. The nano collagen peptide chelate mineral preparation method, according to any one of claims 1 to 3,
wherein the enzyme used in step 1) comprises one or more type selected from a group consisting of collagenase, trypsin, papain, pepsin and alkalase.

5. The nano collagen peptide chelate mineral preparation method, according to any one of claims 1 to 4,
wherein the hydrolysis reaction at step 1) is performed for 2 to 24 hours at 50°C to 70°C.

6. The nano collagen peptide chelate mineral preparation method, according to any one of claims 1 to 5,
further comprising one or more step selected from a group consisting of refining, sterilizing and drying step.

7. The nano collagen peptide chelate mineral preparation method, according to any one of claims 1 to 6,
wherein the nano collagen peptide chelate mineral is used in food, medicine, quasi-drugs, cosmetics or feed.

8. A nano collagen peptide chelate mineral prepared by the preparation method according to any one of claims 1 to 7, and represented by [Chemical Formula 1]: 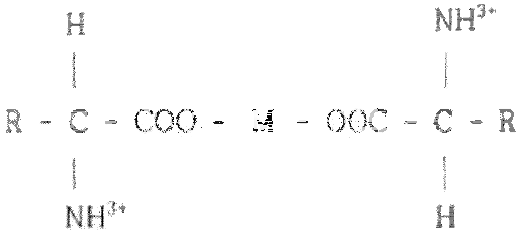 (R representing a side chain that determines a type of amino acid, and M being one or more type selected from a group consisting of Ca, Cu, Zn, Fe, Se, Cr, Mg, Mn and Co.)
